# EUROPÄISCHE PATENTSCHRIFT

(11) **EP 0 857 057 B1**
(45) Veröffentlichungstag und Bekanntmachung des Hinweises auf die Patenterteilung: **06.02.2002**
(21) Anmeldenummer: 96934768.1
(22) Anmeldetag: 21.10.1996
(51) Int. Cl.: A61K 7/38, A61K 7/32

(54) **DESODORIERENDE ZUBEREITUNGEN MIT EINEM GEHALT AN CHITOSAN, ALUMINIUMCHLORHYDRAT UND ESTERASEINHIBITOREN**
DEODORIZING PREPARATIONS CONTAINING CHITOSAN, ALUMINIUM HYDROCHLORATE AND ESTERASE INHIBITORS
PREPARATIONS DESODORISANTES CONTENANT DU CHITOSAN, DU CHLORHYDRATE D'ALUMINIUM ET DES INHIBITEURS D'ESTERASE

(30) Priorität: 28.10.1995 DE 19540296
(43) Veröffentlichungstag der Anmeldung: 12.08.1998
(73) Patentinhaber: Cognis Deutschland GmbH, 40589 Düsseldorf (DE)
(72) Erfinder: WACHTER, Rolf, D-40595 Düsseldorf (DE); LEHMANN, Rudolf, D-42799 Leichlingen (DE); PANZER, Claudia, D-41515 Grevenbroich (DE)
(86) Internationale Anmeldenummer: EP9604563
(87) Internationale Veröffentlichungsnummer: WO9716164

(56) Entgegenhaltungen:
- WO-A-87/06827
- WO-A-91/07165
- LU-A- 71 635
- US-A- 5 411 731
- DATABASE WPI Week 8902 Derwent Publications Ltd., London, GB; AN 89-013338 XP002024547 "Cosmetic with deodorising and antibacterial effect - contains chitosan in the form of its water-soluble acid salt" & JP 63 290 808 A (NITTA GELATIN & EIWA BUSSAN) , 28.November 1988

## Beschreibung

Die Erfindung betrifft desodorierende Zubereitungen mit einem Gehalt an Chitosan, Aluminiumchlorhydrat und Esteraseinhibitoren.

### Stand der Technik

Im Bereich der Körperpflege werden Desodorantien zur Beseitigung störender Körpergerüche eingesetzt. Diese entstehen bei der bakteriellen Zersetzung des an sich geruchlosen Schweißes, insbesondere in den feuchtwarmen Achselhöhlen oder unter ähnlichen, den Mikroorganismen gute Lebensbedingungen bietenden Bedingungen. Körpergerüche können durch geeignete Riechstoffe überdeckt werden. Man kann sie auch bekämpfen, indem man Präparate einsetzt, die die Schweißabsonderung selbst hemmen oder die Zersetzung des Schweißes inhibieren (sogenannte Antihidrotika, Antiperspirantien oder Antitranspirantien). Typische Beispiele für derartige Substanzen sind Aluminiumverbindungen wie Aluminiumsulfat oder Aluminiumchlorhydrat, Zinksalze und Citronensäureverbindungen. Eine Übersicht hierzu findet sich beispielsweise in **Umbach (Hrsg.), "Kosmetik", S.141f., Thieme Verlag, Stuttgart, 1988.**

Aus der täglichen Lebenserfahrung ist jedoch klar, daß das Problem der Geruchsinhibierung, insbesondere bei Hitze oder körperlicher Betätigung keineswegs vollständig gelöst ist. Die Produkte des Marktes vermögen weder die Absonderung von Schweiß noch die Bildung von Gerüchen dauerhaft zu unterbinden. Vielmehr ist die Ihhibierung zeitlich begrenzt und auch davon abhängig, in welchem Umfang Schweiß abgesondert wird. Demzufolge besteht ein andauerndes Bedürfnis nach Produkten, die hinsichtlich der Minimierung der Schweißabsonderung und der Verminderung der Geruchsbildung verbessert sind und dabei gleichzeitig noch eine erhöhte hautkosmetische Verträglichkeit, d.h. ein vermindertes Irritationspotential gegenüber besonders empfindlicher Haut aufweisen. Die Aufgabe der Erfindung hat somit darin bestanden, derartige Produkte zur Verfügung zu stellen.

### Beschreibung der Erfindung

Gegenstand der Erfindung sind desodorierende Zubereitungen, enthaltend
(a) Chitosan,
(b) Aluminiumchlorhydrat und/oder
(c) Esteraseinhibitoren.

Die Verwendung von Aluminiumchlorhydraten und Esteraseinhibitoren vom Typ des Triethylcitrats zur Herstellung von desodorierenden und/oder schweißhemmenden Zusammensetzungen ist aus dem Stand der Technik bekannt. Überraschenderweise wurde gefunden, daß Chitosan, die Aktivität esteraseproduzierender Bakterien inhibieren und zusammen mit den beiden vorgenannten Komponenten eine synergistische desodorierende Wirkung erzielt wird. Chitosan wirkt dabei bakteriostatisch, d.h. die Population der betroffenen Keime wird kontrolliert, jedoch nicht abgetötet, um das biologische Gleichgewicht der Hautflora nicht zu beeinträchtigen. Gleichzeitig führt der Einsatz der kationischen Biopolymere zu einer Verbesserung der hautkosmetischen Verträglichkeit der Produkte.

### Chitosane

Chitosane, die als Komponente (a) in Frage kommen, stellen partiell deacetylierte Chitine unterschiedlichen Molekulargewichtes dar, die den - idealisierten-Monomerbaustein (I) enthalten:

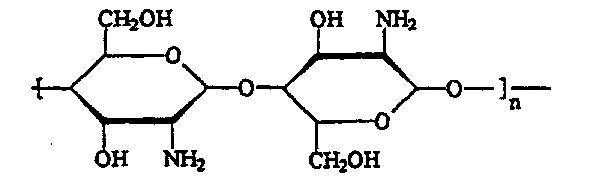

Im Gegensatz zu den meisten Hydrokolloiden, die im Bereich biologischer pH-Werte negativ geladen sind, stellen die bevorzugt einzusetzenden Chitosane unter diesen Bedingungen kationische Verbindungen dar. Die positiv geladenen Chitosane können mit entgegengesetzt geladenen Oberflächen in Wechselwirkung treten und werden daher üblicherweise in kosmetischen Haar- und Körperpflegemitteln eingesetzt. Übersichten zu diesem Thema sind beispielsweise von B.Gesslein et al. in **HAPPI 27, 57** (**1990**), O.Skaugrud in Drug Cosm. Ind. **148, 24(1991)** und E.Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Bevorzugt sind solche Chitosane, die einem nachträglichen Abbau mit Wasserstoffperoxid unterworfen worden sind. Entsprechende Verfahren zur Herstellung von - mikrokristallinem - Chitosan sind beispielsweise in der **WO 91/05808** (Firextra Oy) und der **EP-B1 0 382 150** (Hoechst) beschrieben.

### Aluminiumchlorhydrat

Bei Aluminiumchlorhydaten der Komponente (b) handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über ein Zusammenziehen bzw. Verkleben der Schweißdrüsen durch Eiweißfällung und/oder Feuchtigkeitsentzug [vgl. **J. Soc. Cosm. Chem. 24, 281** (**1973**)]. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J. Pharm. Pharmacol. 26,531 (1975)].**

### Esteraseinhibitoren

Beim Vorhandensein von Schweiß im Achselbereich werden durch Bakterien Enzyme-Esterasen - aktiviert und in den extracellulären Raum abgegeben, die Ester spalten und dadurch Geruchsstoffe freisetzen. Esteraseinhibitoren der Komponente (c), vorzugsweise Trialkylcitrate wie Trimethylcitrat, Tripropylcitrat, Tributylcitrat und insbesondere Triethyl-citrat (Hydagen® CAT, Henkel KGaA, Düsseldorf/FRG) inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme blockiert werden.

Weitere Stoffe, die als Estereseinhibtoren in Betracht kommen, sind Dicarbonsäuren und deren Ester, wie beispielsweise Glutarsäure, Glutarsäuremonoethylester, Glutarsäurediethyl-ester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird.

### Gewerbliche Anwendbarkeit

Kationische Biopolymere haben sich für den beschriebenen Anwendungszweck als bakteriostatisch erwiesen. Ein weiterer Gegenstand der Erfindung betrifft daher ihre Verwendung, alleine oder in Abmischung mit Aluminiumchlorhydraten und/oder Esteraseinhibitoren, zur Herstellung von desodorierenden Zubereitungen.

Die erfindungsgemäßen Zusammensetzungen können in einer bevorzugten Ausführungsform der Erfindung die Komponenten (a), (b) und (c) vorzugsweise in den folgenden Mengenbezogen auf den Feststoffanteil - enthalten:
(a) 0,01 bis 50, vorzugsweise 2 bis 5 Gew.-% Chitosan,
(b) 1 bis 50, vorzugsweise 10 bis 50 Gew.-% Aluminiumchlorhydrat und
(c) 0,01 bis 20, vorzugsweise 1 bis 5 Gew.-% Esteraseinhibitoren.

Hierbei besteht die Maßgabe, daß sich die Mengenangaben zu 100 Gew.-% ergänzen. Die Angaben verstehen sich jeweils auf den Aktivsubstanzgehalt der Komponenten.

### Keimhemmende Mittel

Die erfindungsgemäßen Zubereitungen können als weitere Zusatzstoffe bekannte keimhemmende Mittel enthahen. Typische Beispiele sind Konservierungsmittel mit spezifischer Wirkung gegen grampositive Bakterien wie etwa 2,4,4'-Trichlor-2'-hydroxydiphenylether, Chlorhexidin (1,6-Di-(4-chlorphenylbiguanido)-hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-% - bezogen auf den auf den Feststoffanteil der Zubereitungen.

### Hilfs- und Zusatzstoffe

Um die Wirkstoffe auf eine dosierbare, sparsame, bequeme und kosmetisch ansprechende Weise auf die Haut applizieren zu können, werden sie üblicherweise in Rezepturgrundlagen eingearbeitet. Als wichtigste Grundlagen sind zu nennen: Alkoholische und wäßrig/alkoholische Lösungen, Emulsionen, Gele, Öle, Wachs/Fett-Massen, Stiftpräparate und Puder. So können die erfindungsgemäßen Zubereitungen beispielsweise jeweils bis zu 60 Gew.-% niedere aliphatische Alkohole, vorzugsweise Ethanol sowie organische Säuren wie z.B. Gly-colsäure enthalten. Weitere Einsatzstoffe sind Überfettungsmittel, Emulgatoren, Antioxi-dantien, Talkum, Kieselsäure (z.B. als Träger für das Aluminiumchlorhydrat), sowie Parfumöle, etherische Öle, Farbstoffe und - für Sprayanwendungen - Treibgase wie beispielsweise Propan und/oder Butan. Die Mittel kommen vorzugsweise als Roller (Roll-on-Emulsionen), Stifte, Deo- oder Pumpsprays in den Handel.

### Beispiele

### I. Inhaltsstoffe

A) Kationisches Biopolymer vom Chitosan-Typ (Hydagen® CMF, Henkel KGaA); 1 Gew.-%ig in 0,4 Gew.-%iger wäßriger Glycolsäure
B) Aluminiumchlorhydrat (Locron® L, Hoechst AG)
C) Triethylcitrat (Hydagen® CAT, Henkel KGaA)

### II. Anwendungstechnische Untersuchungen

Die Wirksamkeit der erfindungsgemäßen Mittel wurde stellvertretend über die Esteraseinhibierung bestimmt. Hierzu wurde nach 15 minütiger Einwirkzeit von 2000 ppm der Testgemische auf die Esterase bei pH = 6 deren Restaktivität parallel zu einer ungehemmten Esterase bestimmt (Standard = 100 %). Die Zusammensetzungen R1, R2, R4, R6 und R7 sind erfindungsgemäß, die Rezepturen R3, R5 und R8 dienen zum Vergleich. Die Ergebnisse sind in Tabelle 1 zusammengefaßt (Mengenangaben als Gew.-%).

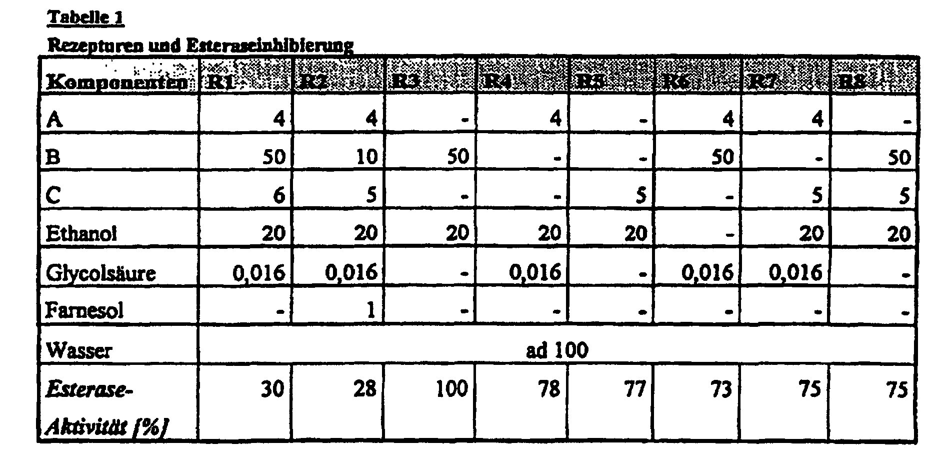

## Patentansprüche

1. Desodorierende Zubereitungen, enthaltend
(a) Chitosan,
(b) Aluminiumchlorhydrat und
(c) weitere Esteraseinhibitoren.

2. Zubereitungen nach Anspruch 1, **dadurch gekennzeichnet, daß** sie als weitere Esteraseinhibitoren Trialkylcitrate enthalten.

3. Zubereitungen nach den Ansprüchen 1 und 2, **dadurch gekennzeichnet, daß** sie - bezogen auf den Feststoffgehalt -
(a) 0,01 bis 5 Gew.-% Chitosan,
(b) 1 bis 50 Gew.-% Aluminiumchlorhydrat und
(c) 0,01 bis 20 Gew.-% Esteraseinhibitoren
enthalten, mit der Maßgabe, daß sich die Mengenangaben zu 100 Gew.-% ergänzen.

## Claims

1. Deodorizing formulations containing
(a) chitosan,
(b) aluminium chlorohydrate and
(c) other esterase inhibitors.

2. Formulations as claimed in claim 1, **characterized in that** they contain contain trialkyl citrates as the other esterase inhibitors.

3. Formulations as claimed in claims 1 and 2, **characterized in that**, based on the solids content, they contain
(a) 0.01 to 5 % by weight of chitosan,
(b) 1 to 50% by weight of aluminium chlorohydrate and
(c) 0.01 to 20% by weight of esterase inhibitors,
with the proviso that the quantities shown add up to 100% by weight.

## Revendications

1. Préparations déodorantes contenant :
(a) du chitosane,
(b) du chlorhydrate d'aluminium et/ou
(c) d'autres inhibiteurs d'estérases.

2. Préparations selon la revendication 1,
**caractérisées en ce qu'**
elles contiennent comme autres inhibiteurs d'estérases des citrates de trialkyle.

3. Préparations selon la revendication 1 ou 2,
**caractérisées en ce qu'**
elles contiennent, par rapport à la teneur en matière solide,
(a) de 0,01 à 5 % en poids de chitosane,
(b) de 1 à 50 % en poids de chlorhydrate d'aluminium, et
(c) de 0,01 à 20 % en poids d'inhibiteurs d'estérases,
étant entendu que les données quantitatives se complètent à 100 % en poids.
